(19) Europäisches Patentamt
European Patent Office
Office européen des brevets

(11) Veröffentlichungsnummer: **0 208 218 B1**

(12) **EUROPÄISCHE PATENTSCHRIFT**

(45) Veröffentlichungstag der Patentschrift: **07.03.90**

(51) Int. Cl.⁴: **C03C 25/02**, B01D 39/20 // G01N33/86

(21) Anmeldenummer: **86108827.6**

(22) Anmeldetag: **28.06.86**

(54) **Gerinnungsneutrale, hydrophile Glasfasern.**

(30) Priorität: **04.07.85 DE 3523969**

(43) Veröffentlichungstag der Anmeldung: **14.01.87 Patentblatt 87/3**

(45) Bekanntmachung des Hinweises auf die Patenterteilung: **07.03.90 Patentblatt 90/10**

(84) Benannte Vertragsstaaten: **AT BE CH DE FR GB IT LI LU NL SE**

(56) Entgegenhaltungen:
**EP-A- 0 080 908**
**FR-A- 2 105 264**
**FR-A- 2 193 026**
**GB-A- 2 014 727**

(73) Patentinhaber: **BOEHRINGER MANNHEIM GMBH, Sandhofer Strasse 116, D-6800 Mannheim 31(DE)**

(72) Erfinder: **Wielinger, Hans, Dr. phil., Im Langgewann 7, D-6940 Weinheim(DE)**
Erfinder: **Freitag, Helmut, Dr. rer. nat., An der Ziegelhütte 13, D-6940 Weinheim(DE)**

EP 0 208 218 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der EP-A 0 045 476 werden Mittel und Verfahren zum Abtrennen von Plasma oder Serum aus Vollblut beschrieben, die es ermöglichen, Inhaltsstoffe aus Vollblut auf einfache Weise zu bestimmen. Gemäß dieser Literaturstelle läßt sich das Plasma von den Erythrozyten abtrennen, indem man das Vollblut durch eine Schicht aus Glasfasern mit Faserdurchmessern von unter 2,5 μ laufen läßt, welche die Erythrozyten zurückhält. Vorzugsweise wird dabei das Blut auf ein Ende eines rechteckigen Glasfaservlieses aufgetragen, von wo aus das Plasma kapillar in den übrigen Bereich transportiert wird. Auf diesen mit Plasma gefüllten Vliesteil wird nach dem Plasmagewinnungsvorgang eine die für die Reaktion notwendigen Reagenzien enthaltende Matrix (Papier, saugfähige Filme etc.) gedrückt, in der die Nachweisreaktion für den nachzuweisenden Parameter über Remissionsmessungen durchgeführt wird.

Dieses einfache Plasmagewinnungs- und Plasmatransport-System ist für alle klinisch-chemisch wichtigen Parameter einsetzbar, mit Ausnahme von Parametern, die im Rahmen der Untersuchungen bei hämostasiologischen Fragestellungen zu bestimmen sind. Es ist bekannt, daß Gerinnungsanalysen prinzipiell in Kunststoffgefäßen oder Glasgefäßen, die durch einen Belag aus Silikonharz inaktiviert sind, durchzuführen sind, weil unbehandeltes Glas die Gerinnbarkeit von Blut oder Plasma beeinflußt. So verkürzt Glas durch Aktivierung die Einphasen-Gerinnungszeit nach Quick von Plasmen, deren Gerinnungsfaktoren im Normalbereich liegen. Die Einphasen-Gerinnungszeit nach Quick wird bei niederprozentigen Plasmen durch Inaktivierung von Gerinnungsfaktoren verlängert. Glas inaktiviert dabei vor allem die Faktoren V und IIa. Durch diese Inaktivierung und damit fälschlich verlängerte Gerinnungszeiten wird der diagnostische Nutzen zunichte gemacht, weil die Verhältnisse der einzelnen Gerinnungsfaktoren verschoben werden. Der in Prozent angegebene Quick-Wert umfaßt neben der Fibrinogenkonzentration die Aktivität der Faktoren II, V, VII und X. Ein Poolplasma von gesunden Spendern wird als 100 % Plasma definiert, über Verdünnung mit physiologischer Kochsalzlösung werden entsprechend niederprozentige Plasmen hergestellt. Über diese Verdünnungsreihe wird eine Bezugskurve erstellt, anhand derer die Quick-Werte für Patientenplasmen ermittelt werden.

Aber auch die Bestimmung der partiellen Thromboplastinzeit (PTT) wird durch die Inaktivierung der Faktoren XII und XI negativ beeinflußt. Der Nachweis von Antithrombin III und Heparin wird durch eine Inaktivierung von Thrombin durch Glas erheblich gestört.

Aufgabe der vorliegenden Erfindung war es daher, die Abtrenn- und Transport-Systeme gemäß EP-A 00 45 476 so zu modifizieren, daß diese gerinnungsneutral werden, ohne ihre Abtrenn- und Transporteigenschaften zu ändern und dadurch auf für die hämostasiologischen Untersuchungen brauchbar werden.

Vordergründig boten sich folgende Lösungsmöglichkeiten: Einsatz von Fasermaterialien aus Kunststoffen. Diese Fasermaterialien haben aber hinsichtlich ihres Abtrenn-Verhaltens gegenüber den Erythrozyten nicht die gewünschten Eigenschaften und beeinflussen die Gerinnbarkeit von Plasmen zum Teil erheblich. Weiter ist bekannt, daß man Gläser durch Silikonisieren mit Silikonharzemulsionen oberflächlich belegen kann und dadurch eine Beeinflussung der Gerinnungsfaktoren ausschließt. Diese Silikonisierung bewirkt im speziellen Fall der Glasfasern jedoch eine so starke Hydrophobierung der Oberfläche, daß keine Benetzung mehr stattfinden kann und somit die Fasern ihre Erythrozyten-Abtrenn- und Plasma-Transporteigenschaften völlig verlieren.

Überraschenderweise wurde gefunden, daß man Glasfaseroberflächen mit bestimmten Silanen chemisch so modifizieren kann, daß sie die hydrophilen Eigenschaften, die z.B. für die Saugfähigkeit von Glasfaser-Vliesen notwendig sind, beibehalten, aber den Einfluß auf die Gerinnungsfaktoren verlieren, d.h. gerinnungsneutral werden.

Durch die Behandlung von Glasfasern mit Verbindungen der allgemeinen Formel I werden unter intermediärer Spaltung der Alkoxygruppen zu Hydroxygruppen und anschließende Reaktion dieser Hydroxygruppen mit den reaktiven Stellen der Glasfaseroberfläche, Glasfasern erhalten, die die oben beschriebenen Eigenschaften haben.

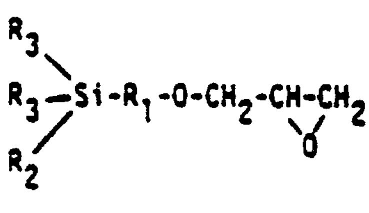

(I)

wobei

$R_1$ = eine Alkylengruppe mit 2–6 C-Atomen

$R_2$ = eine niedere Alkoxygruppe mit 1–6 C-Atomen und

$R_3$ = niedere Alkyl- oder eine Alkoxygruppe mit 1–6 C-Atomen

bedeutet.

Die Behandlung der Glasfasern geschieht in bekannter Art und Weise, indem man in wässrigen, leicht sauer eingestellten Lösungen Substanzen der allgemeinen Formel I einsetzt (FEBS Lett. 93, 5 - 6 (1978)). Bei diesem Vorgang werden sowohl die reaktiven Si-OH-Gruppen ausgebildet, die mit der Glasoberfläche reagieren, als auch die Oxiranringe zu Diolen umgesetzt. Bei Verwendung von Silanen mit einer Alkoxygruppe $R_2$ entstehen monomolekulare Schichten. Bei Verwendung von Silanen mit 2 oder 3 Alkoxygruppen entstehen je nach Reaktionsbedingungen mehrschichtige Überzüge (normalerweise 4 - 12 Schichten), da die Silane auch unter Polykondensation miteinander reagieren.

Eine vorteilhafte Möglichkeit ist die Belegung der Glasoberfläche mit Substanzen der allgemeinen Formel I in wasserfreien organischen Lösungsmitteln, ggf. unter Katalyse mit schwachen Basen, und anschließende Umsetzung der Oxiranringe zu Diolen unter saurer Katalyse in wässriger Lösung (Advan. Chromatogr., 21, 48 - 53 (1978)). Nach diesem Verfahren lassen sich monomolekulare oder wenige Silanmoleküle dicke Schichten aufbauen.

Es ist ferner noch möglich, die Reaktivität der Oxiran- oder der Diolgruppe auszunutzen, um daran weitere Liganden zu knüpfen, die das Glas gerinnungsneutral halten, aber die Hydrophilie verändern, soweit dies notwendig erscheint und den zusätzlichen Aufwand rechtfertigt. Stark ionische Liganden scheiden dabei aus, da sie die Gerinnung beeinflussen, jedoch kann eine Ankuppelung von Aminosäuren und Peptiden vorteilhaft sein. Eine Alkoholyse des Oxiranringes mit einem Monoalkohol führt zu hydrophoberen Glycolmonoethern, mit Polyolen zu hydrophilen Polyolverbindungen.

Das erhaltene Endprodukt hat folgende schematisch dargestellte Struktur:

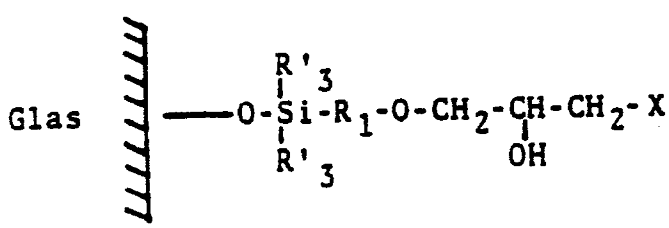

wobei X eine Hydroxygruppe, eine ggf. durch eine oder mehrere Hydroxygruppen substituierte Alkylgruppe, einen Aminosäure oder Peptidrest darstellt und

$R'_3$ entweder eine niedere Alkylgruppe oder eine Sauerstoffbrücke zu den Siliziumatomen der benachbarten Gruppe oder zur Glasoberfläche bedeutet.

So modifizierte Gläser/Glasfasern verhalten sich neutral gegenüber dem Ablauf der Gerinnungskaskade.

Als Alkylengruppen $R_1$ kommen geradkettige oder verzweigte Gruppen mit 2–6 C-Atomen infrage, wobei das Siliziumatom durch mindestens 2C-Atome von dem Ethersauerstoff getrennt sein muß. Ethylen und Propylen werden bevorzugt.

Als Alkyl- bzw. Alkoxygruppen $R_2$ und $R_3$ kommen geradkettige und verzweigte niedere Alkylgruppen mit 1–6 C-Atomen, insesondere Methyl- und Ethylgruppen infrage, die einfach herzustellen und umzusetzen sind. Soweit X eine Alkoxygruppe darstellt, enthält diese ebenfalls 1–6 C-Atome. Methoxy-, Ethoxy-, Propoxy-, 2-Hydroxyethylenoxy-, 2,4-Dihydroxypropylenoxy- sind bevorzugte Gruppen. Als Aminosäure oder Peptidreste kommen insbesondere die im Humanserum enthaltenen natürlichen Aminosäuren und Peptide, insbesondere Albumin infrage, durch die die Gerinnungskaskade nicht gestört wird.

Die erfindungsgemäßen Glasfasern werden nach herkömmlichen Verfahren zu Vliesen verlegt, die, wie in den u.a. Beispielen beschrieben wird, in Mitteln zur Bestimmung von Gerinnungsparametern eingesetzt werden können. Sie können jedoch auch in Form einer kurzen Säule zur Plasmagewinnung gemäß EP-A 0045 476 verwendet werden, worauf das Plasma dann in herkömmlicher Weise in Gerinnungstesten eingesetzt werden kann.

Weitere Ausgestaltungsmöglichkeiten der erfindungsgemäßen Mittel zur Abtrennung von Plasma und zur Verwendung in diagnostischen Mitteln zur Bestimmung von Gerinnungsparametern, können in Analogie zu den in der EP-A 00 45 476 beschriebenen Mitteln, auf die hiermit Bezug genommen wird, hergestellt werden. Es ist selbstverständlich, daß in solchen Mitteln nicht nur die erfindungsgemäßen Glasfaserschichten, sondern auch alle übrigen mit dem Plasma in Kontakt kommenden Teile aus gerinnungsneutralen Materialien bestehen müssen, wobei insbesondere entsprechende, dem Fachmann bekannte Kunststoffe eingesetzt werden.

Beispiel 1

Herstellung von gerinnungsneutralen Glasfasern

Zu 1 Liter Wasser, das mit einer Säure (z.B. Salzsäure oder Essigsäure) auf einen pH von 2,0 - 4,0 vorzugsweise 3,0 gebracht wurde, werden 5 - 50 g vorzugsweise 20 g γ-Glycidoxypropyltrimethoxisilan gegeben. Zur Abspaltung des Methanols und Ausbildung der Si-OH-Gruppen wird solange gerührt, bis

keine Trübung mehr zu sehen ist. Die Reaktion kann durch Erhöhung der Temperatur beschleunigt werden. Besonders bewährt hat sich eine Reaktionszeit von 1 Stunde bei 80° C. Zu dieser Lösung kommen 0,5 - 10 g Glasfasern vorzugsweise 2 -7 g. Es wird 1 bis 4 Stunden vorzugsweise 1 Stunde weiter gerührt, dabei wird das Silan an die Glasoberfläche gebunden und der Oxiranring zur Diolgruppe umgesetzt. Die Reaktion läuft bei höheren Temperaturen schneller ab. Die Glasfasern werden abgesaugt und mit Wasser gründlich nachgewaschen. Die Glasfasern werden in 1 Liter Wasser, das mit Salzsäure auf einen pH von 2,0 - 4,0 vorzugsweise 3,0 eingestellt wurde, aufgeschlämmt und auf einem Blattbildner Blätter mit einem Flächengewicht von 20 - 200 g/m$^2$ gebildet. Das zu wählende Flächengewicht hängt davon ab, zu welcher Verwendung man die gebildeten Vliese benötigt (s. dazu Beispiel).

Beispiel 2

Prüfung der Glasfasern auf Gerinnungsneutralität

Von den gemäß Beispiel 1 hergestellten Glasfasern werden je 40mg mit je 300 µl Poolplasma von gesunden Spendern als auch von mit Antikoagulantien behandelten Patienten zusammengebracht und 1 Minute bei 37° C inkubiert. Danach werden die Plasmen über Zentrifugation abgetrennt. Die Plasmen werden vor und nach der Behandlung mit Glas auf die Einphasen-Gerinnungszeit nach Quick untersucht. Dabei kann der Clotting-Test verwendet werden:
Starten der Gerinnungskaskade mit Calciumchlorid und Thromboplastin und häkeln der Probe, wobei die Zeit bis zur Bildung der Fibrinfäden gemessen wird. Ebenso kann ein photometrischer Test zur Bestimmung der Quickzeit herangezogen werden. (Becher,U. et al.: Neue Aspekte in der Gerinnungsdiagnostik, F.K. Schattauer Verlag, Stuttgart - New York (1984), Seite 17 - 30).

Darüber hinaus wird der Einfluß auf Thrombin (Faktor IIa) wie folgt getestet. 300 µl einer Lösung von Thrombin in Wasser von ca. 3 U/ml wird in der oben beschriebenen Weise mit Glas zusammengebracht. Bei allen Versuchen, bei denen das Glas nach dem erfinderischen Verfahren modifiziert wurde, treten keine Unterschiede im Gerinnungsverhalten von Plasmen vor und nach der Behandlung mit modifiziertem Glas auf. Auch die Thrombinaktivität (Thrombin wird von unbehandeltem Glas völlig inaktiviert) bleibt unverändert.

Die Aktivitätsbestimmung des Thrombins wird mit Tos-Gly-Pro-Arg-p-Nitroanilin als Substrat durchgeführt. Dazu werden 2 ml Puffer Tris/HCl 2,00 mmol/l, pH 8,1 mit 50 µl Thrombinlösung bei 25° C 3 Minuten inkubiert und dann mit 100 µl 3,8 mmol/l Substrat in Puffer die Reaktion gestartet und die Kinetik verfolgt.

Beispiel 3

Bestimmung der Einphasengerinnungszeit nach Quick

Herstellung des Testes

Reagenzmatrix

Ein Papier von einer Flächensaugfähigkeit von 60 - 70 ml/m$^2$ wird mit einer wässrigen Lösung folgender Zusammensetzung imprägniert:
Hepes 250 mmol/l (Puffer)
Tos-Gly-Pro-Arg-p-phenylendiamin 1 mmol/l (Substrat)
N-(4-fluorphenyl)-N-methylaminomethan-phosphonsäure 30 mmol/l (Kuppler)
Kaninchenhirnthromboplastin 1,2 g/l
Diese Lösung wird mit Natronlauge auf pH 7,3 eingestellt.

Nach dem Trocknen werden aus dem imprägnierten Papier Bahnen von 15 mm Breite geschnitten.

Oxidationsmatrix

Ein Nylonnetz, Fadenstärke ca. 40 µm, Maschenweite ca. 60 µm, (Type NY 20 HC Super der Firma Züricher Beuteltuchfabrik, Schweiz), wird mit einer wässrigen Lösung von 50 mmol $K_3[Fe(CN)_6]$/l und 50 mmol Calciumchlorid/l imprägniert. Nach dem Trocknen werden von dem imprägnierten Netz 15 mm breite Bahnen geschnitten.

Testaufbau

Auf eine 100 mm breite Polystyrolfolie werden das erfindungsgemäße Glasfaservlies von einem Flächengewicht von 50 - 60 g/m$^2$ in einer Breite von 15 mm gelegt und mit einem Nylonnetz, mit einer Fadendicke von 140 µm und einer Maschenbreite von 250 µm, das darübergelegt und am Ende angeklebt wird, fixiert (siehe Fig. 1). Am freien Ende des Glasfaservlieses werden die Oxidations- und Reagenzmatrix übereinandergelegt, mit einer 200 µ dicken transparenten Polycarbonatfolie von 15 mm Breite abgedeckt

und festgeklebt (siehe Fig. 1). Die so hergestellten Bänder werden zu 6 mm breiten Streifen geschnitten. Den Aufbau des Teststreifen zeigt die Fig. 1. In dieser stellen dar:

1. Polystyrol-Trägerfolie
2. Kleber
3. Nylonnetz
4. Erfindungsgemäßes Glasfaservlies
5. Oxidationsmatrix
6. Reagenzmatrix
7. Polycarbonatabdeckfolie

Bestimmung der Einphasengerinnungszeit nach Quick

Vergleich zwischen Teststreifen, die mit erfindungsgemäßen Glasfasern hergestellt wurden und nicht silanisierten Glasfasern.

Auf das Nylonnetz, mit dem das Glasfaservlies fixiert ist, werden 35 µl einer Citratblut-Verdünnungsreihe (Hämatokrit um 40 %) von 100 %; 50 %, 33 %, 25 %, 12,5 % Blut in physiologischer NaCl-Lösung pipettiert. Die Erythrozyten werden in dem Abtrennteil zurückgehalten. Die Teststreifen werden dann auf 37° C temperiert und mit einem Remissionsphotometer die Farbbildung nach der Zeit verfolgt. Dabei ist zu beobachten, daß mit den erfindungsgemäßen Glasfasern wesentlich höhere Signale erhalten werden als mit unbehandelten Glasfasern. Als Quick-Zeit kann bei der Verwendung von silanisierten Glasfasern die Zeit genommen werden, innerhalb deren eine Remissionsabnahme von 10 % Remission durchlaufen wird. Hingegen können bei der Verwendung von unbehandelten Glasfasern nur die Zeiten herangezogen werden innerhalb deren eine Änderung um 4 % Remission stattfindet. Auch sind die Streuungen bei Testen mit den erfindungsgemäßen Glasfasern deutlich geringer, da von Seiten des Glases keine störende Beeinflussung auf den Ablauf der Gerinnungskaskade erfolgt. Die Unterschiede werden in der folgenden Tabelle dargestellt.

| Quick % | Test mit erfindungsgemäßen Glasfaservliesen | Test mit nichtbeschichteten Glasfaservliesen | Photometrischer Test mit Citrat-Plasma |
|---|---|---|---|
| | Zeiten in Sek. für eine Remissionsabnahme um 10% | Zeiten in Sek. für eine Remissionsabnahme um 4% | Zeiten in Sek. für eine Extinktionsabnahme um 0,1 E |
| 100% | 36,2 | 27,5 | 33,2 |
| 50% | 44,7 | 48,8 | 45,8 |
| 33% | 53,2 | 71,8 | 57,8 |
| 25% | 61,0 | 94,4 | 67,0 |
| 12,5% | 95,1 | 200,0 | 116,0 |

Aus der Tabelle ist zu ersehen, daß wie von Gerinnungsanalysen mittels Clotting-Tests bekannt, durch Glas bei Plasmen von gesunden Spendern (100% Plasma) eine Aktivierung erfolgt, während niederprozentige Plasmen deutlich inaktiviert werden.

Aus Figur 2, die die Ergebnisse der vorstehenden Tabelle wiedergibt und in der Kurve 1 mit einem Teststreifen gemäß Figur 1 mit nicht beschichtetem Glasfaservlies, Kurve 2 mit einem photometrischen Test und Kurve 3 mit einem Teststreifen gemäß Figur 1 mit erfindungsgemäßem Glasfaservlies erhalten wurden, ist außerdem zu erkennen, daß die Bezugskurve, die mit einem Testaufbau mit den erfindungsgemäßen Glasfasern erhalten wird (3), der des photometrischen Quick-Testes (2) sehr ähnlich ist.

**Patentansprüche**

1. Gerinnungsneutrale, hydrophile Glasfasern, dadurch gekennzeichnet, daß an ihre Oberfläche Silane gemäß folgender Struktur gebunden sind

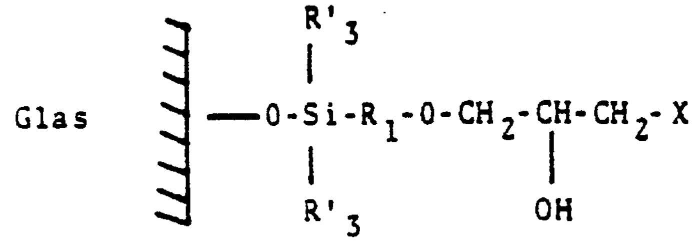

wobei $R_1$ eine Alkylengruppe mit 2–6 C-Atomen, X eine Hydroxygruppe, eine gegebenenfalls durch eine oder mehrere Hydroxygruppen substituierte Alkoxygruppe mit 1–6 C-Atomen, einen Aminosäure- oder Peptidrest darstellt und

$R_3'$ entweder eine niedere Alkylgruppe mit 1–6 C-Atomen oder eine Sauerstoffbrücke zu den Siliziumatomen der benachbarten Gruppe oder zur Glasoberfläche bedeutet.

2. Glasfasern gemäß Anspruch 1, dadurch gekennzeichnet, daß sie an der Glasoberfläche mehrschichtige (1–30, insbesondere 4–12 Molekülschichten) Überzüge aufweisen, wie sie bei Verwendung eines Silans der Formel I

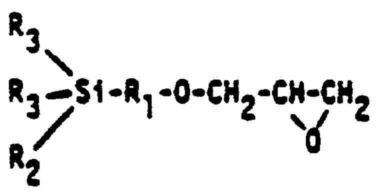
I

worin

$R_1$ eine Alkylengruppe mit 2–6 C-Atomen,

$R_2$ eine niedere Alkoxygruppe mit 1–6 C-Atomen und

$R_3$ eine niedere Alkyl- oder niedere Alkoxygruppe mit 1–6 C-Atomen bedeutet,

mit 2 oder 3 Alkoxygruppen durch Polykondensation der Silane untereinander entstehen.

3. Verfahren zur Herstellung von Glasfasern gemäß Anspruch 1–2, dadurch gekennzeichnet, daß man gereinigte Glasfasern mit einem Silan der Formel I umsetzt

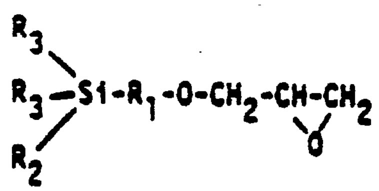
I

worin

$R_1$ eine Alkylengruppe mit 2–6 C-Atomen,

$R_2$ eine niedere Alkoxygruppe mit 1–6 C-Atomen und

$R_3$ eine niedere Alkyl- oder niedere Alkoxygruppe mit 1–6 C-Atomen bedeutet, und durch Hydrolyse, Alkoholyse oder Umsetzung mit einer Aminosäure oder einem Peptid die Oxirangruppe in die Gruppe X überführt.

4. Verfahren nach Anspruch 3, dadurch gekennzeichnet, daß die Umsetzung in wässriger Lösung in Anwesenheit eines sauren Katalysators durchgeführt wird und als Gruppe X eine Hydroxygruppe erhalten wird.

5. Verfahren nach Anspruch 3, dadurch gekennzeichnet, daß die Umsetzung in einem wasserfreien Medium durchgeführt wird.

6. Mittel zur Abtrennung von Plasma aus Blut für hämostasiologische Teste, bestehend aus einer Schicht von Glasfasern mit einem mittleren Durchmesser von 0,2–5 μ, vorzugsweise 0,5–2,5 μ und einer Dichte der Schicht von 0,1–0,5 g/cm³, dadurch gekennzeichnet, daß die Glasfasern eine Silanschicht gemäß Anspruch 1 oder 2 aufweisen.

7. Mittel gemäß Anspruch 6, dadurch gekennzeichnet, daß die Fasern in einer Säule geschichtet sind, deren Kopf für die Aufgabe von Blut und deren Ende für die Abnahme von Plasma vorgesehen ist.

8. Mittel gemäß Anspruch 6, dadurch gekennzeichnet, daß die Glasfaserschicht aus einem Glasfaserpapier oder -vlies besteht und zum Nachweis von Gerinnungsparametern Teil eines diagnostischen Mittels ist.

9. Mittel gemäß Anspruch 8, dadurch gekennzeichnet, daß die Glasfaserschicht die oberste Schicht eines mehrschichtigen diagnostischen Mittels ist.

10. Mittel gemäß Anspruch 8 oder 9, dadurch gekennzeichnet, daß die Glasfaserschicht mit einer Plasmatransportschicht in saugfähigem Kontakt steht, die ihrerseits in saugfähigem Kontakt mit einer oder mehreren Reagenzschichten steht oder mit diesen in Kontakt gebracht werden kann.

11. Verwendung von Glasfasern gemäß Anspruch 1 oder 2 zur Herstellung von Testen für hämostasiologische Untersuchungen.

## Claims

1. Coagulation-neutral, hydrophilic glass fibres, characterised in that on their surfaces are bound silanes according to the following structure

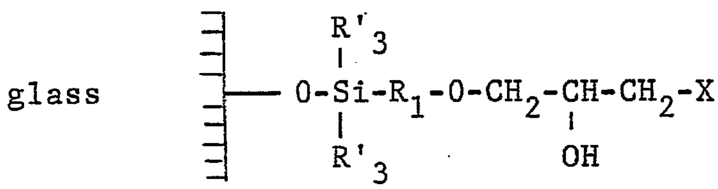

whereby $R_1$ represents an alkylene group with 2 - 6 C-atoms, X a hydroxyl group, an alkoxy group with 1 - 6 C-atoms possibly substituted by one or more hydroxyl groups, an amino acid or peptide residue and $R'_3$ signifies either a lower alkyl group with 1 - 6 C-atoms or an oxygen bridge to the silicon atom of the neighbouring group or to the glass surface.

2. Glass fibres according to claim 1, characterised in that on the glass surface they have multi-layer (1 - 30, especially 4 - 12 molecule layers) coatings as they result in the case of the use of a silane of the formula I

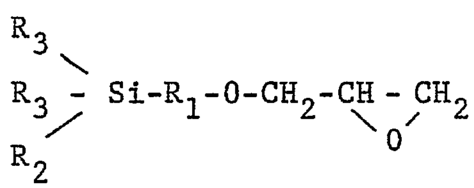

(I)

wherein $R_1$ signifies an alkylene group with 2 - 6 C-atoms, $R_2$ a lower alkoxy group with 1 - 6 C-atoms and $R_3$ a lower alkyl or lower alkoxy group with 1 - 6 C-atoms, with 2 or 3 alkoxy groups by polycondensation of the silanes with one another.

3. Process for the production of glass fibres according to claim 1 - 2, characterised in that one reacts cleaned glass fibres with a silane of the formula I

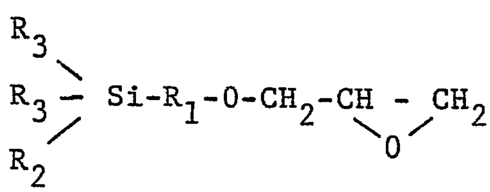

(I)

wherein $R_1$ signifies an alkylene group with 2 - 6 C-atoms, $R_2$ a lower alkoxy group with 1 - 6 C-atoms and $R_3$ a lower alkyl or lower alkoxy group with 1 - 6 C-atoms, and converts the oxiran group into the group X by hydrolysis, alcoholysis or reaction with an amino acid or a peptide.

4. Process according to claim 3, characterised in that the reaction is carried out in aqueous solution in the presence of an acidic catalyst and a hydroxyl group is obtained as group X.

5. Process according to claim 3, characterised in that the reaction is carried out in an anhydrous medium.

6. Agent for the separation of plasma from blood for haemostasiological tests, consisting of a layer of glass fibres with an average diameter of 0.2 - 5 μ, preferably 0.5 - 2.5 μ, and a density of the layer of 0.1 to 0.5 g./cm³, characterised in that the glass fibres have a silane layer according to claim 1 or 2.

7. Agent according to claim 6, characterised in that the fibres are layered in a column, the head of which is provided for the application of blood and the end of which for the removal of plasma.

8. Agent according to claim 6, characterised in that the glass fibre layer consists of a glass fibre paper or fleece and is part of a diagnostic agent for the detection of coagulation parameters.

9. Agent according to claim 8, characterised in that the glass fibre layer is the uppermost layer of a multilayer diagnostic agent.

10. Agent according to claim 8 or 9, characterised in that the glass fibre layer is in absorbent contact with a plasma transport layer which, in turn, is in absorbent contact with one or more reagent layers or can be brought into contact with these.

11. Use of glass fibres according to claim 1 or 2 for the production of tests for haemostasiological investigations.

**Revendications**

1. Fibres de verre hydrophiles, neutres au point de vue de la coagulation, caractérisées en ce qu'à leur surface sont liés des silanes ayant la structure suivante

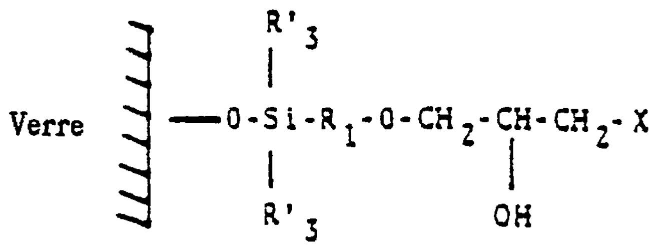

où $R_1$ représente un groupe alkylène comportant 2–6 atomes de carbone, X représente un groupe hydroxy, un groupe alcoxy comportant 1–6 atomes de carbone, éventuellement substitué par un ou plusieurs groupes hydroxy, un reste d'acide aminé ou de peptide et

$R_3$' représente soit un groupe alkyle inférieur comportant 1–6 atomes de carbone, soit un pont oxygène vers l'atome de silicium du groupe voisin ou vers la surface de verre.

2. Fibres de verre selon la revendication 1, caractérisées en ce qu'elles présentent, à la surface de verre, un revêtement multicouches (1–30, en particulier 4–12 couches moléculaires), comme il s'en forme par utilisation d'un silane de formule I

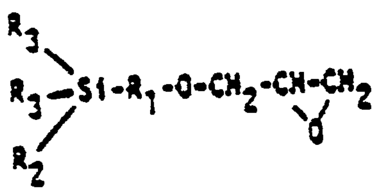

I

où

$R_1$ représente un groupe alkylène comportant 2–6 atomes de carbone,

$R_2$ représente un groupe alcoxy inférieur comportant 1–6 atomes de carbone, et

$R_3$ représente un groupe alkyle inférieur ou alcoxy inférieur comportant 1–6 atomes de carbone,

avec 2 ou 3 groupes alcoxy, par polycondensation des silanes entre eux.

3. Procédé pour la fabrication de fibres de verre selon la revendication 1–2, caractérisé en ce que l'on fait réagir les fibres de verre purifiées, avec un silane de formule I

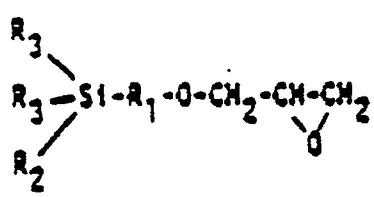

I

où

$R_1$ représente un groupe alkylène comportant 2–6 atomes de carbone,

$R_2$ représente un groupe alcoxy inférieur comportant 1–6 atomes de carbone, et

$R_3$ représente un groupe alkyle inférieur ou alcoxy inférieur comportant 1–6 atomes de carbone,

et l'on transforme le groupe oxyrane, par hydrolyse, alcoolyse ou réaction avec un acide aminé ou un peptide, en le groupe X.

4. Procédé selon la revendication 3, caractérisé en ce que la réaction est effectuée en solution aqueuse, en présence d'un catalyseur acide et que comme groupe X, on obtient un groupe hydroxy.

5. Procédé selon la revendication 3, caractérisé en ce que la réaction est effectuée dans un milieu anhydre.

6. Agent pour la séparation du plasma à partir du sang pour le test de l'hémostase, constitué d'une couche de fibres de verre ayant un diamètre moyen de 0,2–5 μ, de préférence 0,5–2,5 μ, et ayant une densité de couche de 0,1–0,5 g/cm³, caractérisé en ce que les fibres de verre présentent une couche de silane selon la revendication 1 ou 2.

7. Agent selon la revendication 6, caractérisé en ce que les fibres sont empilées par couches dans une colonne, dont la tête est prévue pour le chargement en sang et dont l'extrémité est prévue pour le recueil du plasma.

8. Agent selon la revendication 6, caractérisé en ce que la couche de fibres de verre est constituée d'une nappe ou papier en fibres de verre et en ce qu'elle constitue une partie d'un agent de diagnostic, pour la détermination de paramètres de coagulations.

9. Agent selon la revendication 8, caractérisé en ce que la couche de fibres de verre constitue la couche supérieure d'un agent de diagnostic multicouches.

10. Agent selon la revendication 8 ou 9, caractérisé en ce que la couche de fibres de verre est en contact d'absorption avec une couche de transport de plasma, qui à son tour est en contact d'absorption avec une ou plusieurs couches de réactifs ou qui peut être amenée en contact avec celles-ci.

11. Utilisation des fibres de verre selon la revendication 1 ou 2, pour la préparation de tests pour des recherches d'hémostase.

Fig. 1

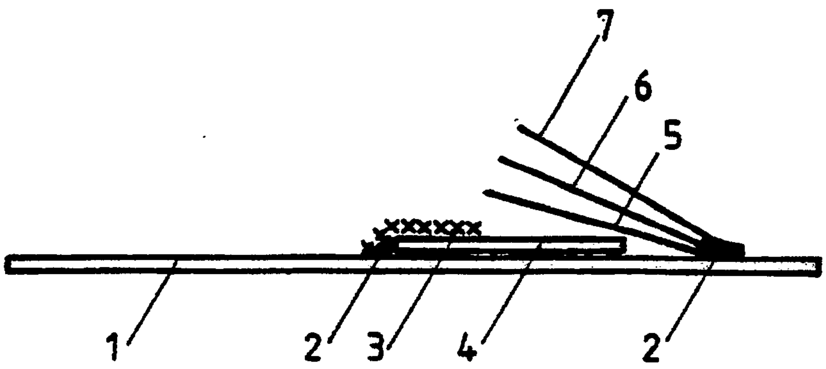
7
6
5
1
2
3
4
2

Fig. 2

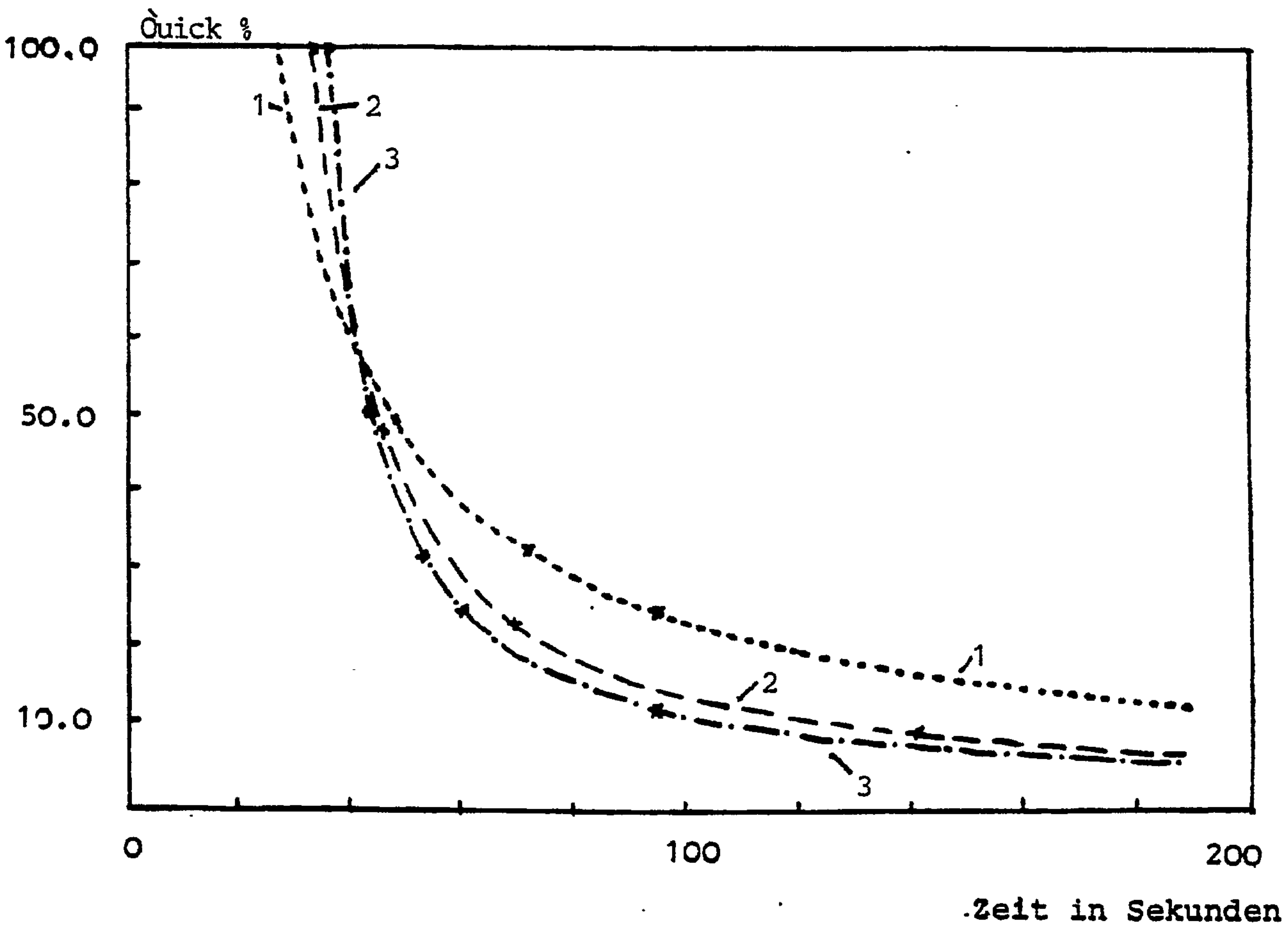
Quick %
100.0
50.0
10.0
1
2
3
1
2
3
0
100
200
Zeit in Sekunden